# EUROPEAN PATENT SPECIFICATION

(11) **EP 4 623 970 B1**
(45) Date of publication and mention of the grant of the patent: **25.11.2026**
(21) Application number: 25164390.4
(22) Date of filing: 18.03.2025
(51) Int. Cl.: A61M 13/00

(54) **APPARATUS AND METHOD FOR TREATING AN INSUFFLATION GAS**
VORRICHTUNG UND VERFAHREN ZUR BEHANDLUNG EINES INSUFFLATIONSGASES
APPAREIL ET PROCÉDÉ DE TRAITEMENT D'UN GAZ D'INSUFFLATION

(30) Priority: 28.03.2024 GB 202404581
(43) Date of publication of application: 01.10.2025
(73) Proprietor: KEYMED (Medical & Industrial Equipment) Limited, Southend-on-Sea Essex SS2 5QH (GB)
(72) Inventor: Wolter, Michael, Southend-on-Sea, SS2 5QH (GB)
(74) Representative: Boult Wade Tennant LLP

(56) References cited:
- DE-B3- 102022 123 989
- US-A1- 2011 230 820
- US-A1- 2013 081 626
- US-A1- 2021 353 897
- US-A1- 2022 088 259

## Description

### Technical Field

The present specification relates to an apparatus and method for treating an insufflation gas.

### Background

It is relatively common during various medical procedures to insufflate regions of the body of human or animal. For example, during laparoscopy the abdomen is typically insufflated with an insufflation gas. The insufflation gas can be any suitable gas, such as carbon dioxide (CO₂).

One of the problems with these procedures is that patients can experience issues such as hypothermia or peritoneal trauma due to increased exposure to cold, dry insufflation gasses. To mitigate this risk, the insufflation gas can be heated and/or humidified. This can be generally described as "treating" the insufflation gas.

Heating of the insufflation gas typically takes place with the use of a resistive wire, for example built into a delivery tube of the insufflator. An electric current is applied to the resistive wire and then this generates heat which heats up the insufflation gas. However, this is not a particularly responsive way of applying heat. If the flow parameters (such as rate) of insufflation gas change, then the delivered insufflation gas may not be heated to the desired temperature.

Humidifying of the insufflation gas is itself typically performed by applying a humidifier liquid (such as water or saline solution) into the insufflation gas stream. For example, a tube set may be filled with a syringe of the humidifier liquid which is then delivered to the insufflation gas flow. However, this requires the flow of insufflation gas to be stopped while the humidifier liquid is inserted.

These systems also require special tubes which incorporate the heating wire and/or the saline inflow which are complex to manufacture.

WO 2004 009166 A1 discloses a device for conditioning an insufflation gas, comprising an inlet for delivering an insufflation gas to a charging device which is used for charging the insufflation gas with a medicament. The insufflation gas which is charged with the medicament flows from the charging device to an outlet and can be fed to an abdominal cavity of a human being or an animal.

There is therefore a need for an improved apparatus and method for treating an insufflation gas.

US 2011 0082416 A1 discloses an insufflator device that connects to a disposable tube set for providing gas to fill an abdominal cavity of a patient to enable surgical procedures.

US 8 147 442 B2 discloses a method and apparatus for treating gas for delivery into a body cavity, body space or body surface of an animal.

US 2021 0322684 A1 discloses a surgical cannula for providing insufflation gases to a surgical cavity of a patient and allowing insertion of medical instruments into the surgical cavity through the cannula that can include a heater within or coupled to the cannula.

US 2022 0233793 A1 discloses a gas heater for a surgical gas delivery system, which includes an elongated tubular body defining an interior flow passage having an inlet port for receiving insufflation gas from a gas source and an outlet port for delivering heated insufflation gas to an insufflation manifold, a dielectric support positioned within the interior flow passage of the tubular body, and a resistive element operatively associated with the dielectric support for heating insufflation gas flowing through the tubular body from the inlet port to the outlet port.

US 9 579 625 B2 discloses a fluid processing assembly that includes a lumen for receiving at least one inlet stream and dispensing a primary product stream, and an energizing device for supplying energy to an energizable portion of the lumen.

US 2021/0243857 A1 discloses apparatuses, systems, and methods for heating a fluid or other material.

WO 2006 131755 A1 discloses heating apparatus that includes radiating means in the form of a microwave source comprising at least one magnetron.

DE 10 2022 123989 B3 discloses, according to its abstract, a respiratory humidifier for a ventilator. The respiratory gas humidifier has a respiratory gas channel that is designed to direct a respiratory gas towards a patient to be ventilated. Furthermore, the respiratory gas humidifier has a nebuliser which is designed to provide an aerosol in the respiratory gas channel, wherein the aerosol comprises at least water. Furthermore, the respiratory gas humidifier has a microwave generator which is connected to the respiratory gas channel via a coupling point in order to evaporate the aerosol by the action of microwave radiation.

US 2021/353897 A1 discloses, according to its abstract, an apparatus used to disinfect viruses/bacteria contaminated exhaust air from devices, such as ventilators, CPAP, APAP, VPAP auto, BiPAO, ECMO, and also devices for air filtration used in cars, buildings, ships, planes, etc.. A heat source is used to burn the contaminated air at elevated temperatures, such as 100° C., 500° C., 1,000° C., and/or even more, in a confined environment to inactivate/destroy viruses/bacteria carried in the air. The heat sources can be, but are not limited to, electrical, gases, infrared, microwave, and Ultraviolet (UV). After disinfection, the exhaust air from the apparatus is then released to the ambient environment, or to the next chamber for further treatment.

US 2013/081626 A1 discloses, according to its abstract, passive heating provided to a flexible tube configured to deliver respiratory gas to a patient. A flexible sheathing body may be removably attached to the flexible tube so as to surround the flexible tube and extend along the flexible tube. A passive heating element integrated with the flexible sheathing body may store non-electrical energy and release the stored non-electrical energy as thermal energy to heat the flexible tube.

### Summary

An apparatus for treating an insufflation gas, according to the present invention, is provided according to claim 1.

This apparatus allows for responsive heating of the insufflation gas, which can be controlled with greater precision.

The heating region of the insufflation tube comprises a microwave susceptor. That is a material which absorbs electromagnetic energy (i.e. microwaves) and converts it to heat. This helps convert the received microwaves into heat for heating the humidified gas flow.

The microwave susceptor may be formed of a material having a greater microwave absorptivity than the humidifier liquid and/or a remainder of the insufflation tube. This means that the susceptor absorbs more of the microwaves and hence generates more heat than the surrounding material, thereby effectively heating the humidified gas flow.

The microwave susceptor may be formed of silicon carbide. Silicon carbide is an effective material for enhancing heating rates. This is because it strongly absorbs microwave energy and subsequently and rapidly transfers the generated thermal energy via conduction.

The microwave susceptor forms at least part of an inner surface of the insufflation tube. This can effectively heat the humidified gas flow.

The nebulizer may be an ultrasonic vaporiser. Such a vaporiser can be beneficial for generating an appropriate mist of humidifier liquid.

The vaporiser may be a piezoelectric nebulizer. Such a vaporiser can be beneficial for generating an appropriate mist of humidifier liquid.

The vaporiser may be configured to generate a mist with an average particle diameter size of no more than 10 µm, preferably the average particle diameter size is between 0.5 µm and 7 µm. A mist with an average particle diameter size in this region is appropriate for insufflation treatment, and effective for the microwave heating.

The apparatus may further comprise one or more sensors arranged to detect one or more of: a temperature of the flow of insufflation gas; a humidity of the flow of insufflation gas; a rate of flow of the insufflation gas; a size of vaporised particles; and/or a speed of vaporised particles. These are various parameters which may be relevant for controlling operation of the apparatus and/or determining how the apparatus is performing.

The one or more sensors may comprise: a first temperature sensor arranged to detect a temperature of the flow of insufflation gas upstream of the heating region; and a second temperature sensor arranged to detect a temperature of the flow of insufflation gas downstream of the heating region. This allows for the heating effect of the microwaves to be clearly identified.

The one or more sensors may comprise a humidity sensor arranged downstream of the vaporiser. The humidity of the delivered gas is another parameter which may be adjusted, and sensing this allows for more precise control.

The apparatus may further comprise a controller arranged to receive reading signals from the one or more sensors, the controller configured to control operation based on the reading signals of one or more of: the microwave generator; the nebuliser; and/or a valve for adjusting the rate of flow of insufflation gas. The controller thus allows for delivery of gas in an ideal range.

The controller may incorporate the use of an artificial intelligence system to control the operation based on the reading signals. There are a number of different inputs and the desired output is a three-dimensional range. Adjusting one input may affect the output in undesired ways. The artificial intelligence system can help mitigate this by learning how the inputs/outputs are interrelated and then making appropriate adjustments to the inputs.

Downstream of the heating region the insufflation tube may comprise a multi-lumen tube, preferably comprising a central lumen for receiving the flow of insufflation gas and one or more annular lumen(s) substantially surrounding the central lumen. This multi-lumen tube can help insulate the insufflation gas to keep it at the desired conditions (such as temperature).

The apparatus may further comprise a reservoir for storing the humidifier liquid, the reservoir in fluid communication with the nebuliser. The separate reservoir allows for the rest of the apparatus to be a closed system which can be replaced.

An insufflator is provided comprising the apparatus disclosed herein. This insufflator allows for responsive heating of the insufflation gas, which can be controlled with greater precision.

An insufflation tube for use with an insufflator apparatus is provided. The insufflation tube comprising: an inlet for receiving a flow of insufflation gas; an outlet for delivering the flow of insufflation gas to an area; and a heating region between the inlet and the outlet, wherein the heating region comprises a microwave susceptor.

This insufflation tube allows for responsive heating of the insufflation gas, which can be controlled with greater precision. A microwave susceptor is a material which absorbs electromagnetic energy (i.e. microwaves) and converts it to heat. This helps convert the received microwaves into heat for heating the humidified gas flow.

The microwave susceptor may be formed of a material having a greater microwave absorptivity than water and/or a remainder of the insufflation tube. This means that the susceptor absorbs more of the microwaves and hence generates more heat than the surrounding material, thereby effectively heating the humidified gas flow.

The microwave susceptor may be formed of silicon carbide. Silicon carbide is an effective material for enhancing heating rates. This is because it strongly absorbs microwave energy and subsequently and rapidly transfers the generated thermal energy via conduction.

The microwave susceptor forms at least part of an inner surface of the insufflation tube. This can effectively heat the humidified gas flow.

The insufflation tube may further comprise a mist region for receiving a mist of humidifier liquid from a nebuliser, the mist region upstream of the heating region. The mist region receives such a mist which is particularly effective for microwave heating.

A method for treating an insufflation gas, according to the present invention, is provided is provided according to claim 10.

This method allows for responsive heating of the insufflation gas, which can be controlled with greater precision.

During the step of delivering the mist of the humidifier liquid the insufflation gas may continue to flow through the insufflation tube. This means that continuous delivery of insufflation gas is achieved and does not need to be halted when greater humidity is desired.

The heating region of the insufflation tube comprises a microwave susceptor. That is a material which absorbs electromagnetic energy (i.e. microwaves) and converts it to heat. This helps convert the received microwaves into heat for heating the humidified gas flow.

The microwave susceptor may be formed of a material having a greater microwave absorptivity than water and/or a remainder of the insufflation tube. This means that the susceptor absorbs more of the microwaves and hence generates more heat than the surrounding material, thereby effectively heating the humidified gas flow.

The microwave susceptor may be formed of silicon carbide. Silicon carbide is an effective material for enhancing heating rates. This is because it strongly absorbs microwave energy and subsequently and rapidly transfers the generated thermal energy via conduction.

The microwave susceptor forms at least part of an inner surface of the insufflation tube. This can effectively heat the humidified gas flow.

The method may further comprise the step of controlling based on one or more reading signals, one or more of: a rate of flow of the insufflation gas; the delivery of the mist of the humidifier liquid; and/or the application of the microwaves. This control allows for delivery of gas in an ideal range.

The method may further comprise the step of generating the one or more reading signals with one or more sensors arranged to detect one or more of: a temperature of the flow of insufflation gas; a humidity of the flow of insufflation gas; a rate of flow of the insufflation gas; a size of vaporised particles; and/or a speed of vaporised particles. These are various parameters which may be relevant for controlling operation of the apparatus.

The step of controlling may incorporate the use of an artificial intelligence system which uses the reading signals. There are a number of different inputs and the desired output is a three-dimensional range. Adjusting one input may affect the output in undesired ways. The artificial intelligence system can help mitigate this by learning how the inputs/outputs are interrelated and then making appropriate adjustments to the inputs.

### Brief Description of the Drawings

The present specification makes reference, by way of example only, to the accompanying drawings in which:
Figure 1 shows a schematic of an apparatus for treating an insufflation gas;
Figure 2 shows a schematic of an apparatus for treating an insufflation gas., incorporated in an insufflator; and
Figure 3 shows another further schematic of an apparatus for treating an insufflation gas.

### Detailed Description

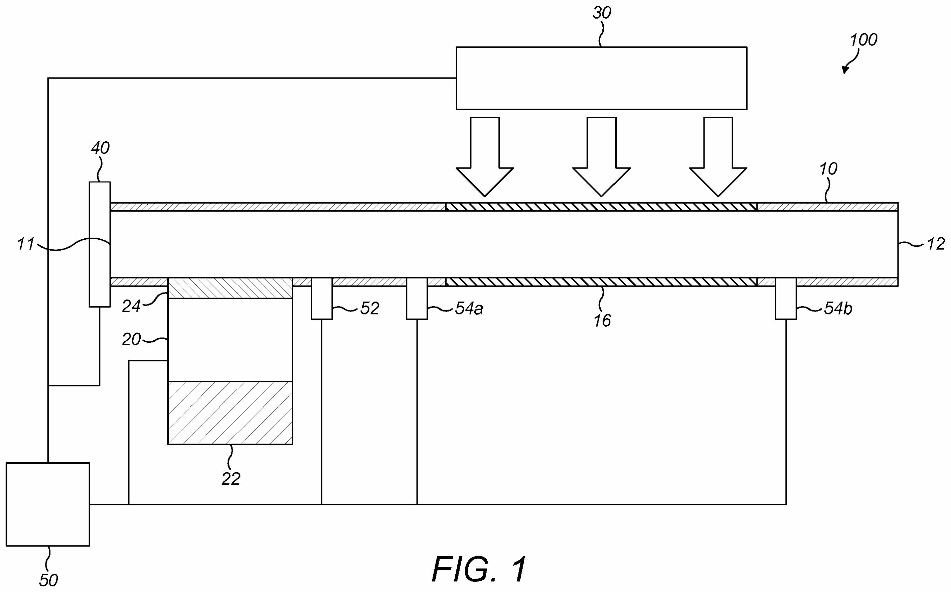

As noted above, each of Figures 1, 2 and 3 show schematics of an apparatus 100 for treating an insufflation gas. Unless otherwise expressly stated to the contrary, any of the following disclosure in relation to an apparatus 100 of one of the Figures is equally applicable to the other apparatuses 100.

The apparatus 100 is suitable for treating an insufflation gas. The treating of the gas could also be referred to as conditioning or processing. In this context, treating an insufflation gas includes one or both of heating the insufflation gas and/or humidifying the insufflation gas. Other treatments may also be performed on the insufflation gas. The insufflation gas can be any suitable gas, but is often CO₂. The apparatus 100 itself may be referred to as an insufflator 200, or alternatively may form a subcomponent of an insufflator 200.

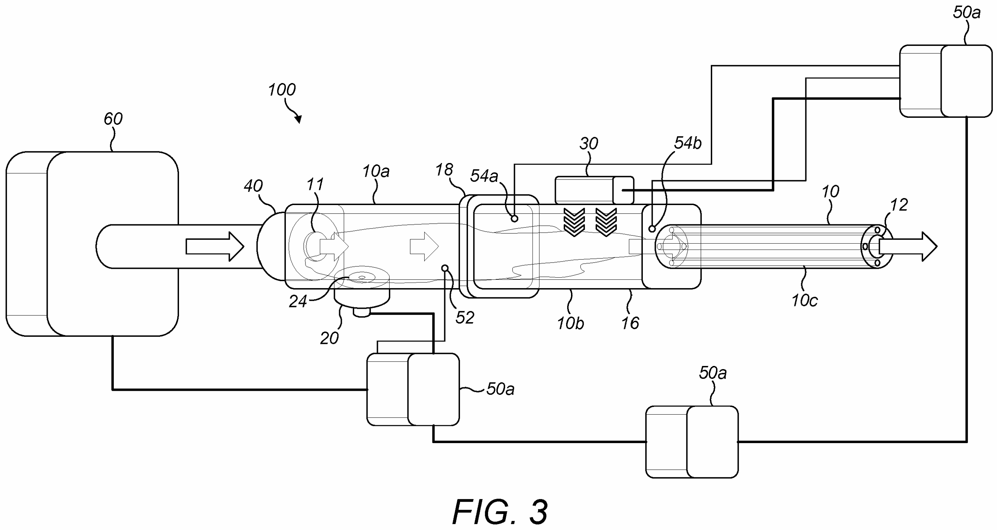

The apparatus 100 comprises an insufflation tube 10. This insufflation tube 10 may be generally cylindrical at least in part, or may be any alternative shapes. For example, Figure 3 shows an insufflation tube 10 which is formed of a number of separate chambers which are generally cuboid. The insufflation tube 10 may be a single continuous tube or may be formed of one or more sections in fluid communication with one another.

The insufflation tube 10 comprises an inlet 11 for a flow of insufflation gas. This inlet 11 may simply be an opening at the end of the insufflation tube 10, for example for connection to a supply of the insufflation gas.

In certain examples, there may be a valve 40 which is arranged to adjust a rate of flow of insufflation gas. This valve 40 may be arranged at or near to the inlet 11 so as to control flow into the inlet 11. The valve can adjust the rate of flow of insufflation gas by changing the degree to which it is open or closed, including fully closed. There may also be a maximum flow rate of insufflation gas when the valve 40 is fully open. Of course, the maximum flow rate of insufflation gas may be defined by characteristics of other components in the apparatus 100.

A nebuliser 20 is provided to deliver a mist of a humidifier liquid 22 into the insufflation tube 10. The insufflation tube 10 may comprise an attachment point for the nebuliser 20, or an intermediate connection, to attach the nebuliser 20 to the insufflation tube 10. As noted above, any humidifier liquid 22 may be used. The area of the insufflation tube 10 where the mist is delivered may be denoted a mist region of the insufflation tube 10. In certain cases, the mist region may be a separate chamber of the insufflation tube 10, such as a separately formed section.

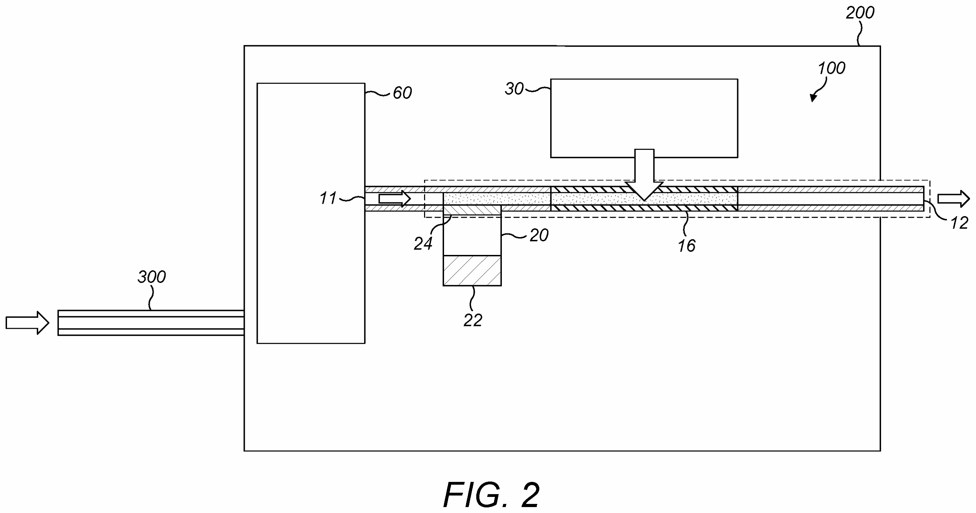

Typically this humidifier liquid 22 may be water or a saline solution. The humidifier liquid 22 is provided in a reservoir as shown in Figures 1 and 2. This reservoir may be attachable and detachable from the nebuliser 20, including when the apparatus 100 is in operation to treat an insufflation gas.

This nebuliser 20 can be any suitable type of nebuliser, including a jet nebuliser, soft mist inhaler, ultrasonic wave nebuliser, and/or vibrating mesh nebuliser.

The nebuliser 20 is arranged or configured to generate a mist with an average particle size such that the particles can be entrained in the insufflation gas flow. For example, the nebulizer 20 may be configured to generate a mist with an average particle diameter size of no more than 10 µm, preferably between 0.5 µm and 7 µm.

In specific examples, the nebuliser 20 is an ultrasonic wave nebuliser (also known as an ultrasonic nebuliser), specifically a piezo nebuliser. In this piezo nebuliser an electronic oscillator generates a high frequency ultrasonic wave. This causes the mechanical vibration of a piezoelectric element, such as a membrane 24. This membrane 24 is in contact with the humidifier liquid 22 (such as the reservoir) and the high frequency vibration produces the vapor mist.

Once the mist of humidifier liquid 22 is delivered to the insufflation tube 10, the mist and flow of insufflation gas may be referred to as a humidified gas flow. The mist of humidifier liquid 22 is entrained in the gas flow.

The apparatus 100 further comprises a microwave generator 30. This microwave generator 30 is arranged to apply microwaves to a heating region of the insufflation tube 10. The heating region may be a conceptual designation of a region of the insufflation tube, or may be a separate section thereof, such as a heating chamber.

This heating region is downstream of the nebuliser 20. That is, the heating region is further from the inlet 11 to the insufflation tube 10 than the nebuliser 20 (or the mist region) is from the inlet 11. This definition of downstream is in relation to the flow of insufflation gas in use.

The microwave generator 30 and generated microwaves heat the humidified gas flow. For example, this may be by heating the entrained humidifier liquid 20 in the humidified gas flow. As a result, the humidified gas flow becomes a heated humidified gas flow. This can be referred to as a treated insufflation gas.

The insufflation tube 10 further comprises an outlet 12 for delivering the heated humidified gas flow to a target area. This may be, for example, an abdominal cavity of a human being or an animal.

In certain examples, the apparatus 100 may further comprise a controller 50, which can also be referred to as a processor, for controlling operation of one or more components of the apparatus 100 as will be described in more detail below. The controller 50 may be a single controller 50 as shown in Figure 1, or a plurality of separate sub-controllers 50a as shown in Figure 3 which collectively act as the controller 50.

The insufflation tube 10 comprises a microwave susceptor 16 in the heating region. A microwave susceptor 16 is a material which absorbs electromagnetic energy (i.e. microwaves) and converts it to heat. Typical microwave susceptors 16 include metallised film, ceramics or metals.

In relative terms, the microwave susceptor 16 may have a greater microwave absorptivity than a material of the rest of the insufflation tube 10. The microwave susceptor 16 may have a greater microwave absorptivity than the humidifier liquid (typically water or saline solution). In other words, the microwave susceptor 16 is provided to improve the conversion of microwaves to heat in the heating region, and thereby heat the humified gas flow more.

A particularly effective material for the microwave susceptor 16 may be silicon carbide (SiC), which is also known as carborundum. This can particularly be formed as a tube. This SiC tube can form all or part of the insufflation tube 10 in the heating region.

The microwave susceptor 16 can form at least a part of an inner surface of the insufflation tube 10 in the heating region. For example, this may be a lining applied to the inner surface. Alternatively, the microwave susceptor 16 itself may be tube-shaped and may form the insufflation tube 10 in this region.

Silicon carbide is an effective material for enhancing heating rates. This is because it strongly absorbs microwave energy and subsequently and rapidly transfers the generated thermal energy via conduction.

In certain examples, the microwave susceptor 16 may be provided on one side of the insufflation tube 10, for example, on a semi-circular (in cross-section) part thereof. This may be a side of the insufflation tube 10 which is farthest away from the microwave generator 30 in use. The insufflation tube 10 further comprises a transmission section with low microwave absorptivity nearer to the microwave generator 30. This means that the microwaves transfer through this transmission section with low microwave absorptivity to the microwave susceptor 16. For example, this transmission section may be generally transparent to microwaves. Collectively, the transmission section and the microwaves susceptor 16 define an entire circumference of the insufflation tube 10 in cross-section.

Figure 3 shows an example where the mist region and the heating region are effectively formed as separate chambers in the insufflation tube 10, with an adaptor 18 therebetween. The adaptor 18 can allow for the change of material in the insufflation tube 10, from a standard tube material such as a plastic in the mist region to the microwave susceptor 16 material in the heating region.

The apparatus 100 may comprise one or more sensors which are used in a control structure. These sensors may be arranged to detect one or more of: a temperature of the flow of insufflation gas; a humidity of the flow of insufflation gas; a rate of flow of the insufflation gas; a size of vaporised particles; and/or a speed of vaporised particles.

For example, the one or more sensors may comprise a temperature sensor. Figure 1 shows an example where there is a first temperature sensor 54a and a second temperature sensor 54b. The first temperature sensor 54a is upstream of the heating region (or at least of the microwave susceptor 16 of the heating region). The second temperature sensor 54b is downstream of the heating region (or at least of the microwave susceptor 16 of the heating region). Each temperature sensor 54a, 54b is arranged to detect a temperature of the flow of insufflation gas in the respective part of the insufflation tube 10. There may also be additional or alternative temperature sensors arranged to detect a temperature of the insufflation gas in other areas, such as the mist region, the inlet 11, and/or the outlet 12.

The one or more sensors may, additionally or alternatively, comprise a humidity sensor 52.

This humidity sensor 52 may be downstream of the mist region and/or nebuliser 20. In certain examples there may be multiple humidity sensors, including one upstream of the mist region and/or nebuliser 20, at the inlet 11, and/or at the outlet 12.

Each of the sensors may be in communication with the controller 50. That means that a reading signal is generated from each sensor and this reading signal is transmitted to the controller 50.

This controller 50 then controls operation of one or more components of the apparatus 100. For example, the controller 50 may control one or more of: the microwave generator 30; the nebuliser 20; and/or the valve 40. This can allow the apparatus 100 to more swiftly and effectively respond to changes in the insufflation gas flow. For example, if the rate of insufflation gas flow increases then the microwaves can be increased in strength/intensity so as to ensure that the greater flow of insufflation gas is still appropriately heated. Likewise, additionally or alternatively, the rate misting of humidifying liquid 22 can be increased.

In further examples, if the second temperature sensor 54b shows that the humidified gas has not been heated to a desired temperature then, again, the controller 50 can adjust other parameters such as the rate of flow (with the valve 40), and/or the strength/intensity of the microwave generator 30.

Alternatively, or additionally, there may be one or more user inputs in communication with the controller 50. One or more of the reading signals could be input by the user in this way, rather than from the one or more sensors.

The controller 50 may incorporate the user of an artificial intelligence (AI) system for the control of the operation of the components of the apparatus 100. This may particularly be useful as for the apparatus 100 there may effectively be an ideal working space which is a three-way parameter of temperature of the insufflation gas, humidity of the insufflation gas, and rate of flow of the insufflation gas. Changing one of the parameters of the apparatus 100 will inherently affect the other two. Having an AI system can mean that the apparatus 100 learns from training data how the parameters change with one another such that, for example, when a flow of gas is increased the system automatically adjusts the nebuliser 20 and/or the microwave generator 30.

Particularly, the AI system may provide a mapping between one or more of the sensed parameters and an estimate of where the gas is in this working space and whether it is in the ideal region. In use, the output gas itself cannot be readily measured since it may be in a sterile environment such as inside an abdominal cavity of a patient.

Training data may be prepared for the AI system by operating the apparatus 100 and measuring directly the output gas. In this training data generation scenario, the sterility of the output gas is not important, since the apparatus 100 can be used purely to generate training data, and not to insufflate a cavity. The measured output temperature of the insufflation gas, humidity of the insufflation gas, and/or rate of flow of the insufflation gas (collectively identified as the output data) may be associated with input data from the one or more sensors (i.e. the reading signals) in the apparatus 100. A model may be created for predicting the measured output data from the reading signals from the one or more sensors. In other words, the model may provide a mapping from the sensor reading signals to the output data.

In use of the apparatus (for example, to insufflate a cavity in a patient's body), the model may be used with input data received from the one or more sensors 30 to provide an estimate of the output parameters of the heated humidified insufflation gas.

Any AI system known in the art may be used for this purpose. For example, in a preferred embodiment, a neural network may be used. The neural network (for example, a multi-layer perceptron) may be trained (for example, by back-propagation or other stochastic gradient descent method) using input data derived from the data provided by the one or more sensors and target data derived from the output data. In some embodiments, the neural network may be a recursive neural network.

The input data may be derived from the data provided by the one or more sensors with or without pre-processing. The pre-processing may involve some form of normalisation. Alternatively, or additionally, the pre-processing may involve a temporal modification such as filtering, for example low-pass filtering. Alternatively, or additionally, the pre-processing may involve a conversion into the frequency domain to produce. The output data may also be pre-processed, either in the same way or in a different way.

The apparatus 100 may use this model to predict how changing one parameter, such as the speed of flow of fluid, will affect the output data and make corresponding adjustments to the operation of components of the apparatus 100, for example by increasing the misting and/or the microwave generation. This is particularly relevant where the output data of the heated humidified insufflation gas is not and/or can not be directly sensed by the one or more sensors.

This AI system may be implemented in software, hardware or a combination thereof.

As explained above, the apparatus 100 may itself form an insufflator 200, or there may be an insufflator 200 which comprises this apparatus 100.

Figure 2 shows an apparatus 100 for treating an insufflation gas which is incorporated in an insufflator 200. As noted above, this apparatus 100 of Figure 2 is generally the same as the apparatus 100 of Figure 1. Unless otherwise expressly stated otherwise, any disclosure in relation to the apparatus 100 of Figure 1 is equally applicable to the apparatus 100 of Figure 2, and vice-versa. This equally applies to the apparatus 100 of Figure 3.

Figure 2 further shows a pressure generator 60 in the insufflator 200. This pressure generator 60 can also be designated as a part of the apparatus 100. The pressure generator 60 may include a pump or other suitable component. The flow of insufflation gas is from the pressure generator 60, into the inlet 11 of the insufflation tube 10. Typically, pressure in a supply reservoir of the insufflation gas (such as a gas bottle, for example a CO₂ bottle) will drive the insufflation gas. The pressure generator 60 may comprise one or more valves or other regulator for controlling a pressure of the insufflation gas. For example, a desired output pressure may be determined and the pressure generator 60 may be adjusted (such as by adjusting valves/regulators) to output insufflation gas with this pressure.

The pressure generator 60 may also be in communication with the controller 50. This means that that controller 50 may receive reading signals from the pressure generator 60 and/or may control operation of the pressure generator 60. For example, the reading signals could include a speed of flow of insufflation gas, a composition of the insufflation gas. Likewise, the pressure generator 60 could be controlled by the controller 50 such as by adjusting a speed of flow of insufflation gas. The control of the pressure generator 60 can therefore be incorporated into the control structures noted above, including the AI control.

The insufflator 200 of Figure 2 also shows a feed 300 for the un-treated insufflator gas (such as CO₂).

Figure 2 shows a number of components inside a box of broken lines. In certain examples, this could include some or all of the features of the apparatus 100. This may exclude, for example, the reservoir of the nebuliser 20. The membrane 24 of the nebuliser 20 may be part of the single-use number of components. In any event, these components may be single-use or dayset components. After a procedure is performed, these components may be detached and replaced. For example, by detaching the apparatus 100 from the pressure generator 60.

In this sense, the insufflation tube 10 itself may also be supplied separately, particularly where it is a single-use or dayset component which can be replaced after a procedure is performed. This insufflation tube 10 is suitable for use with an insufflator 200.

This insufflation tube 10 includes the inlet 11 for receiving the flow in sufflation gas, and the outlet 12 for delivering the flow of insufflation gas to an area. This area may be, for example, an abdominal cavity, or may be an area for further processing or treatment of the insufflation gas. In use, the insufflation gas flows into the inlet 11, through the insufflation tube 10, and out of the outlet 12.

As noted above, the insufflation tube 10 may be a single continuous tube, or may be formed of one or more segments attached to one another. The insufflation tube 10 may be a cylindrical tube, but equally could be any other shape including a square tube. Different sections of the insufflation tube 10 may have different shapes.

The insufflation tube 10 has a heating region (or heating chamber), in which the insufflation gas is to be heated. This insufflation tube 10 has a microwave susceptor 16 in the heating region as described herein. Any disclosure relating to the microwave susceptor 16 in the context of the apparatus 100 is applicable to the microwave susceptor 16 when the insufflation tube 10 is a standalone component.

The insufflation tube 10 may further comprise a mist region (or mist chamber). This mist region, in use, receives a mist of humidifier liquid from the nebuliser 20. This mist region is upstream of the heating region. That is, the insufflation tube 10 is in order: the inlet 11, the mist region, the heating region, then the outlet 12.

In certain examples, the nebuliser 20 or at least a portion thereof attaches to the insufflation tube 10. To facilitate this there may be an attachment region such as a screw fit, or interference fit for the nebuliser 20 to attach to the insufflation tube 10. The insufflation tube 10 may comprise the membrane 24, and the rest of the nebuliser 20 (such as the reservoir) is then attached thereto.

Figure 3 shows the apparatus 100 in a further schematic. As noted above, this apparatus 100 of Figure 3 is generally the same as the apparatus 100 of Figure 1 or Figure 2. Unless otherwise expressly stated otherwise, any disclosure in relation to the apparatus 100 of Figure 1 or Figure 2 is equally applicable to the apparatus 100 of Figure 3, and vice-versa.

The apparatus 100 of Figure 3 particularly shows how the insufflation tube 10 can be formed of different chambers. There is a mist chamber (or mist region) 10a and a heating chamber (or heating region) 10b. These are in fluid connection to one another, such as via an adaptor 18.

Figure 3 also shows how a delivery section 10c of the insufflation tube 10 may be formed as a multi-lumen tube. That is, a tube with a plurality of bores extending therethrough. This delivery section 10c is downstream of the heating chamber 10b (or heating region). In use, the delivery section 10c may deliver the insufflation gas to a region where it is to be used. The delivery section 10c may comprise the outlet 12 of the insufflation tube 10. The multi-lumen tube may comprise a central lumen through which the insufflation gas flows, and one or more annular lumen(s) which substantially surround this central lumen. The annular lumen(s) may be empty, or have a static gas therein, in order to assist with thermally isolating the insufflation gas flow. By substantially surrounding it is noted that the majority of the area around the central lumen is the annular lumen(s), while accounting for support structures or the like.

Figure 3 also shows how in certain examples the controller 50 may be formed of a plurality of sub-controllers 50a in communication with one another. For example, each sub-controller 50a may itself be a separate processor. Each sub-controller 50a may be responsible for controlling separate parts of the apparatus 100.

A method for treating an insufflation gas is also provided. This method may be performed for non-surgical, non-therapeutic and non-diagnostic methods, specifically as the treatment of the gas does not involve any surgical, therapeutic or diagnostic steps - it is entirely performed on a gas.

In the method, an insufflation gas is flowed through an insufflation tube 10. For example, this may be by flowing the insufflation gas into an inlet 11 of the insufflation tube 10. This could be through a feed 300 of an insufflator 200 and then into the insufflation tube 10 and/or via a pressure generator 60.

A mist of humidifier liquid (such as water or saline solution) is then delivered to the insufflation tube 10. This mist may be generated by a nebuliser 20, such as described herein. This mist is then entrained in the insufflation gas flow to crease a humidified gas flow.

Finally, microwaves are applied to a heating region of the insufflation tube 10 in order to heat the humidified gas flow to create a heated humidified gas flow. The microwaves may be generated by a microwave generator 30, such as described herein.

The heated humidified gas flow is then delivered to an area or region where it is needed. For example, this could be for further treatment or could be an abdominal cavity or a colon.

The insufflation tube 10 may be as described herein, either in relation to the insufflation tube 10 itself, or in relation to the assembly 100, or in relation to the insufflator 200. For the avoidance of doubt any disclosure in relation to these components is equally applicable to the insufflation tube 10 of the method. Particularly with reference to the microwave susceptor 16.

The method may further comprise attaching the insufflation tube 10 to an insufflator 200 or to a pressure generator 60. After use, the insufflation tube 10 may be detached therefrom and subsequently disposed of.

The method may further comprise attaching a nebuliser 20 or part thereof, such as those described herein, to the insufflation tube 10. For example, the insufflation tube 10 may comprise the membrane 24, to which the rest of the nebuliser 20 is attached. This rest of the nebuliser 20 or the nebuliser 20 in its entirety is then detached after the insufflation tube 10 has been used, for example prior to disposal of the insufflation tube 10.

The method may further comprise various control steps. These control steps may effectively put into effect the control of the controller 50 discussed herein. For example, one or more reading signals may be taken - such as from one or more sensors. Then, one or more of a rate of flow of the insufflation gas; the delivery of the mist of the humidifier liquid; and/or the application of the microwaves may be controlled. By controlled, it is meant adjusting the relevant components to vary these parameters. The reading signals may be generated by one or more sensors arranged to detect one or more of: a temperature of the flow of insufflation gas; a humidity of the flow of insufflation gas; a rate of flow of the insufflation gas; a size of vaporised particles; and/or a speed of vaporised particles. This may include, for example, the parameters discussed herein.

It is also noted that these control steps of the method can incorporate an artificial intelligence system. This may be the AI system discussed above.

In this sense, an improved apparatus 100, insufflation tube 10 and method is provided.

It will be appreciated that embodiments of the disclosure may be implemented using a variety of different information processing systems. In particular, although the Figures and the discussion thereof provide exemplary computing systems and methods, these are presented merely to provide a useful reference in discussing various aspects of the disclosure. Embodiments may be carried out on any suitable data processing device, such as a personal computer, laptop, tablet, personal digital assistant, mobile telephone, smart phone, set top box, television, server computer, etc.. Of course, the description of the systems and methods has been simplified for purposes of discussion, and they are just one of many different types of systems and methods that may be used. It will be appreciated that the boundaries between logic blocks are merely illustrative and that alternative embodiments may merge logic blocks or elements, or may impose an alternate decomposition of functionality upon various logic blocks or elements.

It will be appreciated that the above-mentioned functionality may be implemented as one or more corresponding modules as hardware and/or software. For example, the above-mentioned functionality may be implemented as one or more software components for execution by a processor of the system. Alternatively, the above-mentioned functionality may be implemented as hardware, such as on one or more field-programmable-gate-arrays (FPGAs), and/or one or more application-specific-integrated-circuits (ASICs), and/or one or more digital-signal-processors (DSPs), and/or other hardware arrangements. Method steps implemented in flowcharts contained herein, or as described above, may each be implemented by corresponding respective modules. Moreover, multiple method steps implemented in flowcharts contained herein, or as described above, may be implemented together by a single module.

It will be appreciated that, insofar as embodiments of the disclosure are implemented by a computer program, then a storage medium and a transmission medium carrying the computer program form aspects of the disclosure. The computer program may have one or more program instructions, or program code, that, when executed by a computer, causes an embodiment of the disclosure to be carried out. The term "program" as used herein, may be a sequence of instructions designed for execution on a computer system, and may include a subroutine, a function, a procedure, a module, an object method, an object implementation, an executable application, an applet, a servlet, source code, object code, a shared library, a dynamic linked library, and/or other sequences of instructions designed for execution on a computer system. The storage medium may be a magnetic disc (such as a hard drive or a floppy disc), an optical disc (such as a CD-ROM, a DVD-ROM or a Blu-ray disc), or a memory (such as a ROM, a RAM, EEPROM, EPROM, Flash memory or a portable/removable memory device), etc.. The transmission medium may be a communications signal, a data broadcast, a communications link between two or more computers, etc..

Each feature disclosed in this specification, unless stated otherwise, may be replaced by alternative features serving the same, equivalent, or similar purpose. Thus, unless stated otherwise, each feature disclosed is one example only of a generic series of equivalent or similar features.

As used herein, including in the claims, unless the context indicates otherwise, singular forms of the terms herein are to be construed as including the plural form and, where the context allows, vice versa. For instance, unless the context indicates otherwise, a singular reference herein including in the claims, such as "a" or "an" (such as a component or an element) means "one or more" (for instance, one or more components, or one or more elements). Throughout the description and claims of this disclosure, the words "comprise", "including", "having" and "contain" and variations of the words, for example "comprising" and "comprises" or similar, mean that the described feature includes the additional features that follow, and are not intended to (and do not) exclude the presence of other components.

The use of any and all examples, or exemplary language ("for instance", "such as", "for example" and like language) provided herein, is intended merely to better illustrate the disclosure and does not indicate a limitation on the scope of the disclosure unless otherwise claimed. No language in the specification should be construed as indicating any non-claimed element as essential to the practice of the disclosure. The present invention is defined by the scope of the attached claims.

Any steps described in this specification may be performed in any order or simultaneously unless stated or the context requires otherwise. Moreover, where a step is described as being performed after a step, this does not preclude intervening steps being performed.

A method of manufacturing and/or operating any of the devices disclosed herein is also provided. The method may comprise steps of providing each of the features disclosed and/or configuring or using the respective feature for its stated function.

## Claims

1. An apparatus (100) for treating an insufflation gas, the apparatus (100) comprising:
an insufflation tube (10) comprising an inlet (11) for receiving a flow of insufflation gas;
a nebuliser (20) arranged to deliver a mist of a humidifier liquid (22) into the insufflation tube (10) for creating a humidified gas flow; and
a microwave generator (30) arranged to apply microwaves to a heating region (10b) of the insufflation tube (10), the heating region (10b) downstream of the nebuliser (20) for heating the humidified gas flow, the heating region (10b) of the insufflation tube (10) comprising a microwave susceptor (16) forming part of an inner surface of the insufflation tube (10),
wherein the insufflation tube (10) comprises a transmission section, the transmission section having a lower microwave absorptivity than the microwave susceptor (16), the transmission section being nearer to the microwave generator (30) than the microwave susceptor (16),
**characterised in that** the transmission section and the microwave susceptor (16) collectively define an entire circumference of the insufflation tube (10) in cross-section.

2. The apparatus (100) of claim 1, wherein the microwave susceptor (16) is formed of:
a material having a greater microwave absorptivity than the humidifier liquid (22) and/or a remainder of the insufflation tube (10);
and/or silicon carbide.

3. The apparatus (100) of any preceding claim, wherein the nebuliser (20) is:
an ultrasonic vaporiser; and/or
a piezoelectric nebuliser.

4. The apparatus (100) of any preceding claim, wherein the nebuliser (20) is configured to generate the mist of the humidifier liquid (22) with an average particle diameter size of no more than 10 µm,
preferably the average particle diameter size is between 0.5 µm and 7 µm.

5. The apparatus (100) of any preceding claim, further comprising one or more sensors (52, 54a, 54b) arranged to detect one or more of:
a temperature of the flow of insufflation gas;
a humidity of the flow of insufflation gas;
a rate of flow of the insufflation gas;
a size of vaporised particles; and/or
a speed of vaporised particles.

6. The apparatus (100) of claim 5, wherein the one or more sensors (52, 54a, 54b) comprises:
a first temperature sensor (54a) arranged to detect a temperature of the flow of insufflation gas upstream of the heating region (10b); and
a second temperature sensor (54b) arranged to detect a temperature of the flow of insufflation gas downstream of the heating region (10b).

7. The apparatus (100) of claim 5 or 6, wherein the one or more sensors (52, 54a, 54b) comprises a humidity sensor (52) arranged downstream of the nebuliser (20).

8. The apparatus (100) of any of claims 5 to 7, further comprising a controller (50, 50a) arranged to receive reading signals from the one or more sensors (52, 54a, 54b), the controller (50, 50a) configured to control operation based on the reading signals of one or more of:
the microwave generator (30);
the nebuliser (20); and/or
a valve (40) for adjusting the rate of flow of insufflation gas,
preferably the controller (50, 50a) incorporates the use of an artificial intelligence system to control the operation based on the reading signals.

9. The apparatus (100) of any preceding claim, wherein downstream of the heating region (10b) the insufflation tube (10) comprises a multi-lumen tube,
preferably comprising a central lumen for receiving the flow of insufflation gas and one or more annular lumen(s) substantially surrounding the central lumen.

10. A method for treating an insufflation gas, the method comprising:
flowing the insufflation gas through an insufflation tube (10);
delivering a mist of a humidifier liquid (22) into the insufflation tube (10) to create a humidified gas flow; and
applying microwaves from a microwave generator (30) to a heating region (10b) of the insufflation tube (10) to heat the humidified gas flow to create a heated humidified gas flow, the heating region (10b) of the insufflation tube (10) comprising a microwave susceptor (16) forming part of an inner surface of the insufflation tube (10),
wherein the insufflation tube (10) comprises a transmission section, the transmission section having a lower microwave absorptivity than the microwave susceptor (16), the transmission section being nearer to the microwave generator (30) than the microwave susceptor (16),
**characterised in that** the transmission section and the microwave susceptor (16) collectively define an entire circumference of the insufflation tube (10) in cross-section.

11. The method of claim 10, wherein during the step of delivering the mist of the humidifier liquid (22) the insufflation gas continues to flow through the insufflation tube (10).

12. The method of claim 10 or 11, wherein the microwave susceptor (16) is formed of:
a material having a greater microwave absorptivity than water and/or a remainder of the insufflation tube (10); and/or
silicon carbide.

13. The method of any of claims 10 to 12, further comprising the step of controlling based on one or more reading signals, one or more of:
a rate of flow of the insufflation gas;
the delivery of the mist of the humidifier liquid (22); and/or
the application of the microwaves,
preferably the method further comprises the step of generating the one or more reading signals with one or more sensors (52, 54a, 54b) arranged to detect one or more of:
a temperature of the flow of insufflation gas;
a humidity of the flow of insufflation gas;
a rate of flow of the insufflation gas;
a size of vaporised particles; and/or
a speed of vaporised particles.

14. The method of claim 13, wherein the step of controlling incorporates the use of an artificial intelligence system which uses the reading signals.

## Patentansprüche

1. Einrichtung (100) zum Behandeln eines Insufflationsgases, wobei die Einrichtung (100) Folgendes umfasst:
ein Insufflationsrohr (10), das einen Einlass (11) zum Aufnehmen eines Insufflationsgasstroms aufweist;
ein Vernebler (20), der so angeordnet ist, dass er einen Nebel einer Befeuchtungsflüssigkeit (22) in das Insufflationsrohr (10) abgibt, um einen befeuchteten Gasstrom zu erzeugen; und
ein Mikrowellengenerator (30), der so angeordnet ist, dass er Mikrowellen auf einen Heizbereich (10b) des Insufflationsrohrs (10) anwendet, wobei sich der Heizbereich (10b) stromabwärts von dem Vernebler (20) zum Erhitzen des befeuchteten Gasstroms befindet, wobei der Heizbereich (10b) des Insufflationsrohrs (10) einen Mikrowellensuszeptor (16) umfasst, der einen Teil einer Innenfläche des Insufflationsrohrs (10) bildet,
wobei das Insufflationsrohr (10) einen Transmissionsabschnitt umfasst, wobei der Transmissionsabschnitt eine geringere Mikrowellenabsorptionsfähigkeit als der Mikrowellensuszeptor (16) aufweist, wobei sich der Transmissionsabschnitt näher am Mikrowellengenerator (30) befindet als der Mikrowellensuszeptor (16),
**dadurch gekennzeichnet, dass**
der Transmissionsabschnitt und der Mikrowellensuszeptor (16) zusammen einen gesamten Umfang des Insufflationsrohrs (10) im Querschnitt bilden.

2. Einrichtung (100) nach Anspruch 1, wobei der Mikrowellensuszeptor (16) aus Folgendem geformt ist:
einem Material, das eine höhere Mikrowellenabsorptionsfähigkeit aufweist als die Befeuchtungsflüssigkeit (22) und/oder ein Rest des Insufflationsrohrs (10);
und/oder Siliciumcarbid.

3. Einrichtung (100) nach einem vorstehenden Anspruch, wobei der Vernebler (20) Folgendes ist:
ein Ultraschallverdampfer und/oder
ein piezoelektrischer Vernebler.

4. Einrichtung (100) nach einem vorstehenden Anspruch, wobei der Vernebler (20) so konfiguriert ist, dass er den Nebel der Befeuchtungsflüssigkeit (22) mit einer mittleren Partikeldurchmessergröße von nicht mehr als 10 µm erzeugt,
wobei vorzugsweise die mittlere Partikeldurchmessergröße zwischen 0,5 µm und 7 µm beträgt.

5. Einrichtung (100) nach einem vorstehenden Anspruch, weiter umfassend einen oder mehrere Sensoren (52, 54a, 54b), die so angeordnet sind, dass sie eines oder mehrere von Folgendem erfassen:
eine Temperatur des Insufflationsgasstroms;
eine Feuchtigkeit des Insufflationsgasstroms;
eine Durchflussrate des Insufflationsgases;
eine Größe verdampfter Partikel; und/oder
eine Geschwindigkeit verdampfter Partikel.

6. Einrichtung (100) nach Anspruch 5, wobei der eine oder die mehreren Sensoren (52, 54a, 54b) Folgendes umfassen:
einen ersten Temperatursensor (54a), der so angeordnet ist, dass er eine Temperatur des Insufflationsgasstroms stromaufwärts von dem Heizbereich (10b) erfasst; und
einen zweiten Temperatursensor (54b), der so angeordnet ist, dass er eine Temperatur des Insufflationsgasstroms stromabwärts von dem Heizbereich (10b) erfasst.

7. Einrichtung (100) nach Anspruch 5 oder 6, wobei der eine oder die mehreren Sensoren (52, 54a, 54b) einen Feuchtigkeitssensor (52) umfassen, der stromabwärts von dem Vernebler (20) angeordnet ist.

8. Einrichtung (100) nach einem der Ansprüche 5 bis 7, weiter umfassend eine Steuereinheit (50, 50a), die zum Empfangen von Lesesignalen von dem einem oder den mehreren Sensoren (52, 54a, 54b) angeordnet ist, wobei die Steuereinheit (50, 50a) so konfiguriert ist, dass sie den Betrieb auf Grundlage der Lesesignale eines oder mehrerer von Folgendem steuert:
dem Mikrowellengenerator (30);
dem Vernebler (20); und/oder
einem Ventil (40) zum Einstellen der Durchflussrate des Insufflationsgases,
wobei die Steuereinheit (50, 50a) vorzugsweise die Verwendung eines Systems künstlicher Intelligenz zum Steuern des Betriebs auf Grundlage der Lesesignale einbezieht.

9. Einrichtung (100) nach einem vorstehenden Anspruch, wobei das Insufflationsrohr (10) stromabwärts von dem Heizbereich (10b) ein Mehrlumenrohr umfasst,
wobei es vorzugsweise ein zentrales Lumen zum Aufnehmen des Isoliergasstroms und ein oder mehrere ringförmige Lumen/Lumina, die das zentrale Lumen im Wesentlichen umgeben, umfasst.

10. Verfahren zum Behandeln eines Insufflationsgases, wobei das Verfahren Folgendes umfasst:
Strömenlassen des Insufflationsgases durch ein Insufflationsrohr (10);
Abgeben eines Nebels einer Befeuchtungsflüssigkeit (22) in das Insufflationsrohr (10), um einen befeuchteten Gasstrom zu erzeugen; und
Anlegen von Mikrowellen aus einem Mikrowellengenerator (30) an einen Heizbereich (10b) des Insufflationsrohrs (10) zum Erhitzen des befeuchteten Gasstroms, um einen erwärmten befeuchteten Gasstrom zu erzeugen, wobei der Heizbereich (10b) des Insufflationsrohrs (10) einen Mikrowellensuszeptor (16) umfasst, der einen Teil einer Innenfläche des Insufflationsrohrs (10) bildet,
wobei das Insufflationsrohr (10) einen Transmissionsabschnitt umfasst, der eine geringere Mikrowellenabsorptionsfähigkeit aufweist als der Mikrowellensuszeptor (16),
wobei sich der Transmissionsabschnitt näher am Mikrowellengenerator (30) befindet als der Mikrowellensuszeptor (16),
**dadurch gekennzeichnet, dass**
der Transmissionsabschnitt und der Mikrowellensuszeptor (16) zusammen einen gesamten Umfang des Insufflationsrohrs (10) im Querschnitt bilden.

11. Verfahren nach Anspruch 10, wobei während des Schritts des Abgebens des Nebels der Befeuchtungsflüssigkeit (22) das Insufflationsgas weiterhin durch das Insufflationsrohr (10) strömt.

12. Verfahren nach Anspruch 10 oder 11,
wobei der Mikrowellensuszeptor (16) aus Folgendem geformt ist:
einem Material, das eine höhere Mikrowellenabsorptionsfähigkeit aufweist als Wasser und/oder ein Rest des Insufflationsrohrs (10); und/oder
Siliciumcarbid.

13. Verfahren nach einem der Ansprüche 10 bis 12, weiter umfassend den Schritt des Steuerns, auf Grundlage eines oder mehrerer Lesesignale, eines oder mehrerer von Folgendem:
einer Durchflussrate des Insufflationsgases;
der Abgabe des Nebels der Befeuchtungsflüssigkeit (22); und/oder
der Anwendung der Mikrowellen,
wobei das Verfahren vorzugsweise weiter den Schritt des Erzeugens des einen oder der mehreren Lesesignale mit einem oder mehreren Sensoren (52, 54a, 54b) umfasst, die so angeordnet sind, dass sie eines oder mehrere von Folgendem erfassen:
eine Temperatur des Insufflationsgasstroms;
eine Feuchtigkeit des Insufflationsgasstroms;
eine Durchflussrate des Insufflationsgases;
eine Größe verdampfter Partikel; und/oder
eine Geschwindigkeit verdampfter Partikel.

14. Verfahren nach Anspruch 13, wobei der Schritt des Steuerns die Verwendung eines Systems künstlicher Intelligenz einbezieht, das die Lesesignale verwendet.

## Revendications

1. Appareil (100) de traitement d'un gaz d'insufflation, l'appareil (100) comprenant :
un tube d'insufflation (10) comprenant une entrée (11) destinée à recevoir un flux de gaz d'insufflation ;
un nébuliseur (20) agencé pour délivrer une brume d'un liquide humidificateur (22) dans le tube d'insufflation (10) afin de créer un flux de gaz humidifié ; et
un générateur de micro-ondes (30) agencé pour appliquer des micro-ondes à une région de chauffage (10b) du tube d'insufflation (10), la région de chauffage (10b) se trouvant en aval du nébuliseur (20) pour chauffer le flux de gaz humidifié, la région de chauffage (10b) du tube d'insufflation (10) comprenant un suscepteur de micro-ondes (16) faisant partie d'une surface intérieure du tube d'insufflation (10),
dans lequel le tube d'insufflation (10) comprend une section de transmission, la section de transmission présentant une absorptivité des micro-ondes inférieure à celle du suscepteur de micro-ondes (16), la section de transmission étant plus proche du générateur de micro-ondes (30) que le suscepteur de micro-ondes (16),
**caractérisé en ce que**
la section de transmission et le suscepteur de micro-ondes (16) définissent collectivement une circonférence entière du tube d'insufflation (10) en coupe transversale.

2. Appareil (100) selon la revendication 1, dans lequel le suscepteur de micro-ondes (16) est constitué :
d'un matériau présentant une absorptivité des micro-ondes supérieure à celle du liquide humidificateur (22) et/ou d'un reste du tube d'insufflation (10) ;
et/ou de carbure de silicium.

3. Appareil (100) selon une quelconque revendication précédente, dans lequel le nébuliseur (20) est :
un vaporisateur à ultrasons ; et/ou
un nébuliseur piézoélectrique.

4. Appareil (100) selon une quelconque revendication précédente, dans lequel le nébuliseur (20) est configuré pour générer la brume du liquide humidificateur (22) avec un diamètre moyen des particules n'excédant pas 10 µm,
de préférence, le diamètre moyen des particules est compris entre 0,5 µm et 7 µm.

5. Appareil (100) selon une quelconque revendication précédente, comprenant en outre un ou plusieurs capteurs (52, 54a, 54b) agencés pour détecter un ou plusieurs parmi :
une température du flux de gaz d'insufflation ;
une humidité du flux de gaz d'insufflation ;
un débit du flux de gaz d'insufflation ;
une taille de particules vaporisées ; et/ou
une vitesse de particules vaporisées.

6. Appareil (100) selon la revendication 5, dans lequel les un ou plusieurs capteurs (52, 54a, 54b) comprennent :
un premier capteur de température (54a) agencé pour détecter une température du flux de gaz d'insufflation en amont de la région de chauffage (10b) ; et
un second capteur de température (54b) agencé pour détecter une température du flux de gaz d'insufflation en aval de la région de chauffage (10b).

7. Appareil (100) selon la revendication 5 ou 6, dans lequel les un ou plusieurs capteurs (52, 54a, 54b) comprennent un capteur d'humidité (52) agencé en aval du nébuliseur (20).

8. Appareil (100) selon l'une quelconque des revendications 5 à 7, comprenant en outre un dispositif de commande (50, 50a) agencé pour recevoir des signaux de lecture provenant des un ou plusieurs capteurs (52, 54a, 54b), le dispositif de commande (50, 50a) étant configuré pour commander le fonctionnement sur la base des signaux de lecture d'un ou plusieurs parmi :
le générateur de micro-ondes (30) ;
le nébuliseur (20) ; et/ou
une vanne (40) pour régler le débit du flux de gaz d'insufflation,
de préférence, le dispositif de commande (50, 50a) intègre l'utilisation d'un système d'intelligence artificielle pour commander le fonctionnement sur la base des signaux de lecture.

9. Appareil (100) selon une quelconque revendication précédente, dans lequel en aval de la région de chauffage (10b), le tube d'insufflation (10) comprend un tube multi-lumières,
comprenant de préférence une lumière centrale destinée à recevoir le flux de gaz d'insufflation et une ou plusieurs lumières annulaires entourant sensiblement la lumière centrale.

10. Procédé de traitement d'un gaz d'insufflation, le procédé comprenant :
la circulation du gaz d'insufflation à travers un tube d'insufflation (10) ;
la délivrance d'une brume d'un liquide humidificateur (22) dans le tube d'insufflation (10) pour créer un flux de gaz humidifié ; et
l'application de micro-ondes provenant d'un générateur de micro-ondes (30) à une région de chauffage (10b) du tube d'insufflation (10) pour chauffer le flux de gaz humidifié afin de créer un flux de gaz humidifié chauffé, la région de chauffage (10b) du tube d'insufflation (10) comprenant un suscepteur de micro-ondes (16) faisant partie d'une surface intérieure du tube d'insufflation (10),
dans lequel le tube d'insufflation (10) comprend une section de transmission, la section de transmission présentant une absorptivité des micro-ondes inférieure à celle du suscepteur de micro-ondes (16),
la section de transmission étant plus proche du générateur de micro-ondes (30) que le suscepteur de micro-ondes (16),
**caractérisé en ce que**
la section de transmission et le suscepteur de micro-ondes (16) définissent collectivement une circonférence entière du tube d'insufflation (10) en coupe transversale.

11. Procédé selon la revendication 10, dans lequel, pendant l'étape de délivrance de la brume du liquide humidificateur (22), le gaz d'insufflation continue de circuler à travers le tube d'insufflation (10).

12. Procédé selon la revendication 10 ou 11,
dans lequel le suscepteur de micro-ondes (16) est constitué :
d'un matériau présentant une absorptivité des micro-ondes supérieure à celle de l'eau et/ou d'un reste du tube d'insufflation (10) ; et/ou
de carbure de silicium.

13. Procédé selon l'une quelconque des revendications 10 à 12, comprenant en outre l'étape de commande, sur la base d'un ou plusieurs signaux de lecture, d'un ou plusieurs parmi :
un débit du flux de gaz d'insufflation ;
la délivrance de la brume du liquide humidificateur (22) ; et/ou
l'application des micro-ondes,
de préférence, le procédé comprend en outre l'étape de génération des un ou plusieurs signaux de lecture à l'aide d'un ou plusieurs capteurs (52, 54a, 54b) agencés pour détecter un ou plusieurs parmi :
une température du flux de gaz d'insufflation ;
une humidité du flux de gaz d'insufflation ;
un débit du flux de gaz d'insufflation ;
une taille de particules vaporisées ; et/ou
une vitesse de particules vaporisées.

14. Procédé selon la revendication 13, dans lequel l'étape de commande intègre l'utilisation d'un système d'intelligence artificielle qui utilise les signaux de lecture.
